(19) Europäisches Patentamt / European Patent Office / Office européen des brevets

(11) **EP 2 874 664 B1**

(12) **FASCICULE DE BREVET EUROPEEN**

(45) Date de publication et mention de la délivrance du brevet:
**19.06.2019 Bulletin 2019/25**

(51) Int Cl.:
*A61K 51/04* (2006.01)    *A61K 51/12* (2006.01)
*A61P 35/00* (2006.01)    *A61K 103/30* (2006.01)
*A61K 103/32* (2006.01)

(21) Numéro de dépôt: **13739670.1**

(22) Date de dépôt: **17.07.2013**

(86) Numéro de dépôt international:
**PCT/EP2013/065131**

(87) Numéro de publication internationale:
**WO 2014/012997 (23.01.2014 Gazette 2014/04)**

(54) **COMPOSITION À USAGE THÉRAPEUTIQUE COMPRENANT UNE PHASE ORGANIQUE LIPIDIQUE ET UN COMPLEXE DE TERRE RARE RADIOISOTOPIQUE**

ZUSAMMENSETZUNG ZUR THERAPEUTISCHEN VERWENDUNG, MIT EINER ORGANISCHEN LIPIDPHASE UND EINEM KOMPLEX VON EINEM RADIOISOTOP DER SELTENEN ERDEN

COMPOSITION FOR THERAPEUTIC USE, INCLUDING AN ORGANIC LIPID PHASE AND A RARE-EARTH RADIOISOTOPE COMPLEX

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.07.2012 FR 1256951**

(43) Date de publication de la demande:
**27.05.2015 Bulletin 2015/22**

(73) Titulaires:
- **Centre Eugene Marquis**
  **35000 Rennes (FR)**
- **Ecole Nationale Superieure de Chimie**
  **35700 Rennes (FR)**
- **Université de Rennes I**
  **35000 Rennes Cedex (FR)**

(72) Inventeurs:
- **LEPAREUR, Nicolas**
  **35770 Vern-sur-Seiche (FR)**
- **GARIN, Etienne**
  **35520 La Meziere (FR)**
- **NOIRET, Nicolas**
  **35250 Saint-Sulpice-la-Foret (FR)**
- **ARDISSON, Valérie**
  **35000 Rennes (FR)**

(74) Mandataire: **Vidon Brevets & Stratégie**
  **16B, rue de Jouanet**
  **BP 90333**
  **35703 Rennes Cedex 7 (FR)**

(56) Documents cités:
**WO-A1-2011/101436**

- **URIZZI P ET AL: "Attachment of radiometals to LDL with lipid analogues of EDTA or DTPA type complexing agents. Comparison of two labeling procedures", JOURNAL DE CHIMIE PHYSIQUE, SOCIETE DE CHIMIE PHYSIQUE, PARIS, FR, vol. 94, no. 2, 1 janvier 1997 (1997-01-01), pages 371-375, XP008089471, ISSN: 0021-7689**
- **YU J ET AL: "Y-oxine-ethiodol, a potential radiopharmaceutical for the treatment of liver cancer", APPLIED RADIATION AND ISOTOPES, ELSEVIER, OXFORD, GB, vol. 58, no. 5, 1 mai 2003 (2003-05-01), pages 567-573, XP027388557, ISSN: 0969-8043 [extrait le 2003-05-01] cité dans la demande**
- **SUBRAMANIAN SURESH ET AL: "Preparation of Lu-177-Labeled Oxine in Lipiodol as a Possible Agent for Therapy of Hepatocellular Carcinoma: A Preliminary Animal Study", CANCER BIOTHERAPY & RADIOPHARMACEUTICALS, vol. 25, no. 5, octobre 2010 (2010-10), pages 539-543, XP002693466, ISSN: 1084-9785 cité dans la demande**

- **DAS TAPAS ET AL: "Preparation of 166Ho-oxine-lipiodol and its preliminary bioevaluation for the potential application in therapy of liver cancer.", NUCLEAR MEDICINE COMMUNICATIONS MAY 2009, vol. 30, no. 5, mai 2009 (2009-05), pages 362-367, XP008160641, ISSN: 0143-3636 cité dans la demande**

- **JIANLIN Y ET AL: "Raman spectroscopic studies on tropolone complexes with La, Nd, Sm, Yb", SPECTROCHIMICA ACTA. PART A: MOLECULAR AND BIOMOLECULAR SPECTROSCOPY, ELSEVIER, AMSTERDAM, NL, vol. 64, no. 4, 1 juillet 2006 (2006-07-01), pages 1072-1076, XP028036481, ISSN: 1386-1425, DOI: 10.1016/J.SAA.2005.09.017 [extrait le 2006-07-01]**

## Description

### 1. Domaine de l'invention

Le domaine de l'invention est celui de la formulation de compositions à usage thérapeutique chez l'homme.

Plus précisément, l'invention concerne une composition comprenant une phase organique lipidique et un complexe de terre rare radioisotopique.

### 2. Art antérieur

Le cancer du foie chez l'homme est une pathologie grave qui représente le cinquième cancer le plus commun dans le monde. Son incidence varie grandement selon les régions du monde. Il affecte plus particulièrement l'Asie du Sud-Est et l'Afrique, mais son incidence dans les pays développés, et particulièrement l'Europe, est en forte progression (E. Garin, P. Bourguet, in Ell and Gambir 3rd Ed., Nuclear Medicine in Clinical Diagnosis and Treatment, Hong-Kong: Churchill-Livingstone, 2004, 473-483).

Son pronostic est extrêmement faible. Les possibilités thérapeutiques sont limitées par le degré de sévérité de l'hépatopathie sous-jacente et par l'extension intra-hépatique de la maladie. La transplantation hépatique est le seul traitement réellement curatif traitant la tumeur et la maladie hépatique favorisante. La résection chirurgicale est également une méthode chirurgicale efficace. Elle n'est malheureusement applicable qu'à des patients non cirrhotiques, ce qui représente moins de 5 % des cas dans les pays occidentaux. Dans ce contexte, deux approches thérapeutiques semblent les plus prometteuses : la radiothérapie métabolique par voie intra-artérielle ou intra-tumorale et la chimioembolisation artérielle.

La radiothérapie métabolique est largement utilisée dans le traitement, curatif ou palliatif, de nombreux cancers, et notamment du cancer du foie. La radiothérapie métabolique des tumeurs hépatiques se fait principalement *via* l'artère hépatique. Le foie sain est irrigué en sang et oxygène à la fois par la veine porte et l'artère hépatique. Néanmoins, dans le cas des tumeurs, ces dernières, richement vascularisées, sont essentiellement irriguées par l'artère hépatique, tandis que l'irrigation des tissus sains se fait à 80 % par la veine porte.

La chimioembolisation artérielle a été développée pour améliorer les performances de la radiothérapie métabolique. Cette technique consiste à injecter dans l'artère hépatique du patient un mélange contenant une substance chimiothérapeutique associée à des particules qui provoquent une embolie, c'est-à-dire qu'elles bloquent le flux sanguin vers la tumeur.

Cette thérapie alternative à la résection chirurgicale et à la radiothérapie présente un double avantage :

- elle permet de libérer une dose très concentrée de substance chimiothérapeutique dans la tumeur en épargnant les tissus sains et le reste de l'organisme,
- l'embolie de la tumeur permet d'y bloquer le médicament, ce qui augmente son efficacité, tout en affaiblissant la tumeur par la privation de nutriments et d'oxygène.

De plus, les effets secondaires pour le patient sont grandement limités et cette thérapie semble donner un avantage en termes de survie aux patients traités par cette voie (B. Lambert, J.Nucl.Med., 2005, 46, 60-66).

Le vecteur le plus utilisé pour l'administration intra-hépatique est le Lipiodol. Le Lipiodol, également connu sous le nom d'Ethiodol® aux Etats Unis, est un agent de contraste liposoluble obtenu par iodation et estérification de l'huile d'oeillette (mélange d'acides linoléique, oléique, palmitique et stéarique), huile obtenue à base de graines de pavot. La proportion d'iode est d'environ 38 % en masse (soit 475 mg/mL). Le Lipiodol est sélectivement capté par les hépatocarcinomes (CHC) et par certaines métastases hépatiques, d'origine colonique, neuroendocrine et mammaire. Le Lipiodol a donc été utilisé pour la détection de l'hépatocarcinome et de ses éventuelles tumeurs satellites non détectables par les méthodes usuelles d'imagerie, puis également pour véhiculer des substances chimiothérapeutiques. Il a également été montré un temps de rétention intra-tumoral largement supérieur au temps de rétention dans le foie sain, avec une rétention dans l'hépatocarcinome pouvant atteindre plusieurs mois. La persistance du Lipiodol au sein des tumeurs a amené à la proposition d'un marquage covalent de celui-ci par de l'iode-131 radioactif ($^{131}$I) afin d'effectuer une radiothérapie ciblée, l'iode « froid », c'est-à-dire non radioactif, contenu dans le Lipiodol étant remplacé par de l'iode radioactif. Cependant, la durée de vie de l'iode-131 ($^{131}$I) n'est que de 8 jours et cet élément est la source d'importantes émissions de rayonnements $\gamma$. Par conséquent, l'intérêt pour le développer en thérapie clinique de routine a rapidement décliné.

Par ailleurs, les propriétés de l'iode-131 étant sub-optimales, d'autres radioéléments, avec des caractéristiques plus adaptées, ont été proposés, notamment l'yttrium-90 ($^{90}$Y) et le rhénium-186 ou 188 ($^{186}$Re, $^{188}$Re). Toutefois, l'yttrium-90 est coûteux et difficile à imager, et des risques de relargage, conduisant à une irradiation médullaire indésirable, ne sont pas à négliger, ce qui limite son développement (S.J. Wang et al., J. Nucl. Med., 1996, 37, 332-335).

En ce qui concerne l'yttrium, deux stratégies de marquage ont été décrites. La première, similaire au marquage à l'Iode-131, consiste en un marquage covalent du Lipiodol (S.J. Wang et al., J. Nucl. Med., 1996, 37, 332-335.), *via* un chélate EDTB (N,N,N',N'-tetrakis(2-benzymidazoylmethyl)1,2-ethanediamine). Cependant, cette technique s'est avérée peu pratique et non reproductible.

La deuxième stratégie, actuellement la voie la plus développée, consiste en la solubilisation dans le Lipiodol d'un complexe chimiquement neutre et lipophile d'yttrium. D'après la littérature, deux complexes lipophiles ont été décrits pour marquer le Lipiodol, 1'8-hydroxyquinoline ou oxine (J. Yu et al., Appl. Radiat. Isot., 2003, 58, 567-573.) et l'acide di(2-ethylhexyl) orthophosphorique ou P204 (P.Y. Mu et al., J. Radioanal. Nucl. Chem., 2007, 272, 669-671.). Concernant le complexe P204, aucune information quant à sa structure, sa biodistribution, sa toxicité et sa stabilité n'est pour le moment disponible. Des complexes lipophiles de lutétium et d'holmium à base d'oxine ont également été décrits (S. Subramanian et al., Cancer Bioth. Radiopharm., 2010, 25, 539-543 ; T. Das et al., Nucl. Med. Commun., 2009, 30, 362-367). Toutefois, les complexes à base d'oxine sont très instables : les ions terre rare se détachent très rapidement du complexe oxine pour se fixer majoritairement sur les os.

En outre, la préparation de radiotraceurs, et en particulier le marquage du Lipiodol, entraîne une irradiation non négligeable du personnel manipulant. Dans ce contexte, plusieurs systèmes automatisés ont été décrits dans la littérature, principalement pour la préparation de radiotraceurs fluorés et carbonés, pour l'imagerie par Tomographie par Emission de Positons (TEP), mais également pour la préparation de traceurs rhéniés dont l'activité est supérieure à 15 GBq (S.J. Oh et al., Appl. Radiat. Isot., 2001, 54, 419-427 ; S.J. Oh et al., Appl. Radiat. Isot., 2003, 59, 225-230).

L'article URIZZI et al. : « Attachement of radiometals to LDL with lipid analogues of EDTA or DTPA type complexing agents. Comparison of two labeling procédures » (JOURNAL DE CHIMIE PHYSIQUE, vol. 94, no. 2, 1 janvier 1997, pages 371-375, XP008089471, ISSN : 0021-789) compare deux protocoles de fixation d'un radioélément (M= 111In, 153Gd) sur les LDL par l'intermédiaire d'un agent complexant lipophile (L).

L'article JIANLIN Y et al : « Raman spectroscopic studies on tropolone complexes with La, Nd, Sm, Yb « (SPECTROCHIMICA ACTA. PART A : MOLECULAR AND BIOMOLECULAR SPECTROSCOPY, vol. 64, no. 4, 1 juillet 2006, pages 1072-1076, XP028036481, ISSN : 1386-1425, DOI : 10.1016/J.SAA.2005.06.017) décrit la préparation de différents complexes de tropolone avec différents métaux lanthanides (La, Nd, Sm et Yb) dans une solution non-aqueuse par oxydation électrochimique directe.

Dans ce contexte, nous avons développé un nouveau complexe lipophile de terre rare radioisotopique permettant de marquer aisément le Lipiodol, à base de tropolone ou d'un dérivé de tropolone.

## 3. Objectifs de l'invention

L'invention a notamment pour objectif de pallier ces inconvénients de l'art antérieur.

Plus précisément, un objectif de l'invention est de fournir, dans au moins un mode de réalisation, un complexe lipophile radiomarqué stable.

Un autre objectif de l'invention est de mettre en oeuvre, dans au moins un mode de réalisation, une méthode de fabrication de ces complexes qui permette de réduire les risques de radiation pour le manipulateur.

L'invention a encore pour objectif de fournir, dans au moins un mode de réalisation, un complexe lipophile permettant de fournir une composition permettant de traiter le cancer du foie chez l'humain.

## 4. Exposé de l'invention

Ces objectifs, ainsi que d'autres qui apparaîtront par la suite, sont atteints à l'aide d'une composition thérapeutique comprenant un complexe de formule (1) suivante comprenant une terre rare radioisotopique, ledit complexe étant solubilisé dans une phase organique lipophile, ladite phase organique étant le Lipiodol®:

$$[M(L)_3] \qquad (1)$$

dans laquelle :

- M désigne la terre rare radioisotopique $^{90}$Y, et
- L désigne un ligand tropolone ou un ligand dérivé de la tropolone choisi parmi une halogénotropolone, l'$\alpha$-methyltropolone, la $\beta$-méthyltropolone, la $\gamma$-méthyltropolone, l'$\alpha$-isopropyltropolone, la $\beta$-isopropyltropolone, la $\gamma$-isopropyltropolone, la nootkatine, l'acide stipitatique, l'acide pubérrulique, l'acide pubérulonique, la purpurogalline, la colchicéine ou un dérivé de ceux-ci, L désignant de préférence la $\beta$-isopropyltropolone.

Ainsi, l'invention repose sur une approche tout à fait nouvelle et originale de combiner les propriétés des radioisotopes de terres rares à une phase organique lipophile, ladite phase organique permettant de véhiculer ces terres rares radioisotopiques auprès des cellules pathologiques. Ainsi, les thérapies par chimioembolisation utilisant cette composition sont plus efficaces. Les terres rares sont complexées sous forme ionique au ligand, permettant le marquage de la phase organique lipophile. Or, les complexes composés d'une terre rare radioisotopique sous forme ionique et d'un ligand tropolone ou dérivé de la tropolone sont particulièrement stables.

La phase organique lipophile peut-être un solvant organique ou une huile. Dans le cas où la phase organique est une huile, elle sera préférentiellement un mélange d'esters d'acides gras iodés.

La terre rare utilisée dans le cadre de l'invention, $^{90}$Y présente des propriétés particulièrement intéressantes pour le traitement des tumeurs.

Selon l'invention, ladite phase organique lipophile est le Lipiodol®. Le Lipiodol est un mélange d'esters méthyliques iodés de l'huile d'oeillette, huile produite à base de graine de pavot. L'utilisation du Lipiodol dans une composition selon la présente invention permet d'obtenir une composition stable. De plus, elle est caractérisée par une fixation tumorale préférentielle et durable, permettant ainsi de traiter efficacement le cancer du foie.

Dans un mode de réalisation avantageux, le Lipiodol est mis en émulsion dans une phase aqueuse, de préférence ladite phase aqueuse étant du sérum physiologique. Ceci permet d'améliorer sa biodistribution et de formuler des compositions injectables bien tolérées par l'humain.

L'invention a également pour objet un procédé de fabrication comprenant les étapes de :

    a. synthèse en une étape du complexe de formule (1),
    b. purification du complexe de formule (1) sur colonne,
    c. évaporation du solvant organique à une température comprise entre 40°C et 100°C,
    d. filtration stérilisante,
    e. solubilisation du complexe de formule (1) dans ladite phase organique lipophile.

La composition selon l'invention peut être aisément préparée de manière manuelle. Toutefois, la composition selon l'invention comprenant un complexe de terre rare radioisotopique de formule (1) et la phase organique lipophile peut également être préparée à l'aide d'un système automatisé. L'automatisation du procédé de synthèse selon l'invention permet de limiter l'irradiation des manipulateurs par la radioactivité tout en permettant de produire la composition selon l'invention de manière reproductible et avec un bon rendement.

Avantageusement, la synthèse en une étape du complexe de formule (1) se fait à partir d'un précurseur ayant la formule (2) suivante :

$$MZ_3 \qquad (2)$$

dans laquelle M désigne la terre rare radioisotopique $^{90}$Y et Z est un anion halogénure, un anion nitrate, un anion triflate ou un anion acétate.

La synthèse en une étape consiste à mélanger sous agitation le complexe $MZ_3$ à une solution de ligand sous agitation, à température ambiante. Un solvant permettant d'éliminer l'anion Z est ensuite ajouté au mélange. Après une centrifugation ou séparation liquide-solide sur colonne, la phase contenant d'un côté le solvant et le complexe $[M(L)_3]$ et de l'autre, la phase aqueuse contenant l'anion Z, sont séparées.

La purification s'effectue par chromatographie, notamment par extraction liquide-solide (SPE). La purification sur colonne peut se faire sur une colonne C8 ou encore sur une colonne C18. On entend par colonne C8 ou C18 des colonnes de type Sep-Pak® en phase inverse dont la phase stationnaire est une phase apolaire constituée de gel de silice sur lequel sont greffées des chaînes de 8 ou 18 atomes de Carbone. En ce cas, l'élution se fait en utilisant une phase mobile polaire et se choisit en fonction de la nature chimique des molécules à éluer. Le solvant résiduel est évaporé par chauffage à une température comprise entre 40°C et 100°C.

Après élution, le complexe purifié subit une filtration stérilisante avant d'être solubilisé dans 2 à 3 ml de Lipiodol non radioactif. On entend par filtration stérilisante le passage du complexe en solution à travers un filtre de porosité $0,2\,\mu m$ pour éliminer les micro-organismes pathogènes.

L'automatisation du procédé rend possible une utilisation de cette formulation à des fins cliniques et pharmaceutiques en limitant grandement l'exposition du manipulateur à la radioactivité. Ainsi, le procédé selon la présente invention permet d'obtenir, par rapport à l'art antérieur, des compositions stables, efficaces et compatibles avec une utilisation thérapeutique concrète.

Dans un mode de réalisation avantageux, le procédé selon l'invention comprend en outre une étape finale de nanoencapsulation. Ainsi, la composition selon l'invention peut être administrée par voie orale ou injectable. La nature de la capsule dépend de la cible physiologique à atteindre. De plus, il est possible de greffer, sur la surface des nanocapsules, des anticorps ou des peptides spécifiques des tumeurs afin d'en améliorer le ciblage. Par exemple, il est possible de greffer des analogues de la somatostatine pour le traitement des tumeurs neuroendocrines. La composition selon l'invention, une fois encapsulée, trouve également une application dans le traitement des gliomes.

Un autre aspect de l'invention concerne l'utilisation de la composition pour le traitement du cancer du foie chez l'humain. La composition est caractérisée par une fixation tumorale préférentielle et durable, permettant ainsi de traiter efficacement le cancer du foie. Elle permet donc de traiter différentes formes de cancer du foie, quelle que soit leur étiologie ou leur histologie (hépatocarcinome, cholangiocarcinome, metastases hépatiques...).

**5. Description d'un mode de réalisation de l'invention**

Le principe général de l'invention repose sur le marquage d'une phase organique lipophile par un complexe organique portant un ion terre rare radioisotopique, la phase organique lipophile étant un vecteur de choix pour amener le complexe radioisotopique auprès des cellules cancéreuses et le marqueur radioisotopique permettant de détruire spécifiquement les cellules pathologiques.

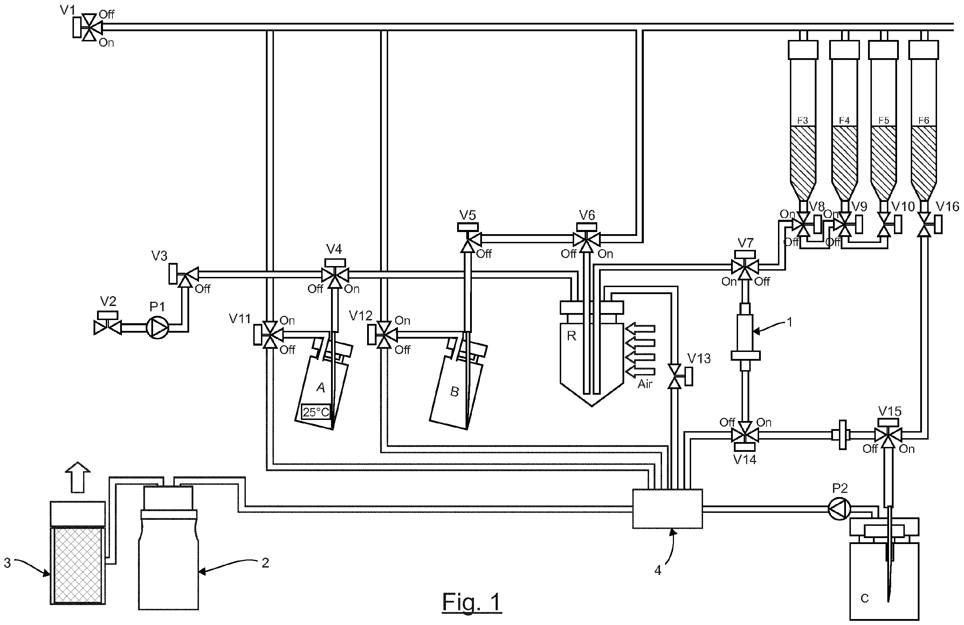

Les modes de réalisation suivant sont donnés à titre d'exemples de la présente invention et n'en constitue nullement une limitation. La figure 1 représente le schéma du procédé automatisé.

Exemple 1 : Préparation du complexe $^{90}$Y-Tropolone par extraction selon la méthode manuelle

On mélange dans un récipient adapté 1 mL de chlorure d'yttrium-90 ($^{90}$Y), présentant une radioactivité de 0,8 mCi, à 1 mL d'une solution de tropolone à $10^{-2}$ mol/L dans du tampon PBS (Phosphate Buffered Saline, à pH 7,4). Après 5 min d'agitation à température ambiante, 2 mL de chloroforme sont ajoutés et les phases sont séparées par centrifugation. La phase organique est collectée.

Le rendement de marquage en pourcentage est calculé de la façon suivante :

$$\text{Rendement de marquage } (\%) = [\text{activité de la phase lipophile (Bq)} \times 100] \,/$$

$$[\text{activité de la phase lipophile (Bq)} + \text{activité de la phase aqueuse (Bq)}]$$

La pureté radiochimique compatible avec une application pharmaceutique se définit comme au moins 90% de complexe $M(L)_3$ contenus dans la phase lipophile.

L'analyse de chromatographie sur couche mince sur papier Whatman a pour objectif de déterminer la pureté radiochimique de la solution préparée. L'éluant utilisé pour effectuer la migration est le méthanol. La chromatographie est ensuite effectuée selon les méthodes bien connues de l'homme de l'art. Brièvement, une goutte de la solution radiomarquée est déposée sur une bandelette CCM, qui est ensuite placée dans une chambre de développement. Un solvant, ici le méthanol, est disposé dans le réservoir sans toucher le point de dépôt. La chambre est refermée et la migration du front de solvant s'opère, entraînant avec lui l'échantillon de solution radiomarquée. A la fin de la migration, la bandelette est déposée pour révélation et visualisation sur une plaque de phosphoimageur, par exemple à l'aide du phosphoimageur Cyclone de Perkin-Elmer.

La Pureté Radiochimique (notée PRC) est exprimée en % et calculée de la façon suivante :

$$\text{PRC} = [\text{activité du spot radioactif d'intérêt (Bq)} \times 100] \,/\, [\text{activité totale (Bq)}]$$

$$\text{Le ratio de migration de la radioactivité Rf} = (\text{radioactivité du front de migration du}$$

$$\text{solvant entraînant le composé (Bq))} \,/\, (\text{radioactivité totale de la bande CCM (Bq))}$$

Dans cet exemple, le rendement (Rdt) est égal à 89,5 % et la pureté radiochimique (PRC) est de 96 %.

Exemple 2 : Préparation du complexe $^{90}$Y-β-isopropyltropolone par extraction selon la méthode manuelle

On mélange dans un récipient adapté 0,5 mL d'acétate d'yttrium-90 ($^{90}$Y) ayant une radioactivité de 1,63 mCi, à 0,5 mL d'une solution de β-isopropyltropolone à $10^{-2}$ mol/L dans l'éthanol. Après 5 min d'agitation à température ambiante, 2 mL de chloroforme sont ajoutés et les phases sont séparées par centrifugation. La phase organique est collectée.

Rendement (Rdt) = 98,3 %

Pureté radiochimique (PRC) = 99,9 %

Exemple 3 : Préparation du complexe $^{90}$Y-Tropolone selon la méthode manuelle

On mélange dans un récipient adapté 1 mL de chlorure d'yttrium-90 ayant une radioactivité de 1,05 mCi, à 1 mL d'une solution de tropolone à $10^{-2}$ mol/L dans le PBS (pH = 7,4). Après 5 min d'agitation à température ambiante, la solution est purifiée sur deux colonnes Sep-Pak® C$_{18}$ (préalablement activées par sérum physiologique), et le complexe est élué par 2 mL d'éthanol.
Rendement (Rdt) = 70 %
Pureté radiochimique (PRC) = 95,1 %

Exemple 4 : Préparation du complexe $^{90}$Y-β- isopropyltropolone selon la méthode manuelle

On mélange dans un récipient adapté 0,5 mL d'acétate d'yttrium-90 ayant une radioactivité de 1,4 mCi, à 0,5 mL d'une solution de β-isopropyltropolone à $10^{-2}$ mol/L dans l'éthanol. Après 5 min d'agitation à température ambiante, la solution est purifiée sur cartouche phase inverse Sep-Pak® C8 par 5 mL d'eau distillée. Le complexe $^{90}$Y-β-isopropyltropolone est élué de la cartouche par 2 mL d'éthanol.
Rendement (Rdt) = 75 %
Pureté radiochimique (PRC) = 92,7 %

Exemple 5 : Préparation du composé marqué au $^{90}$Y en solution dans le Lipiodol

La solution organique obtenue dans les exemples 1 à 4 est évaporée à une température comprise entre 40°C et 100°C, et le résidu repris par 2 mL de Lipiodol. Le mélange est agité 5 minutes. La phase lipiodolée radiomarquée est recueillie.

Exemple 6 : Préparation du composé marqué au $^{90}$Y en solution dans le Labrafac™ CC

La solution organique obtenue dans les exemples 1 à 4 est évaporée à une température comprise entre 40°C et 100°C, et le résidu repris par 2 mL de Labrafac™ CC (caprylic-capric acid triglycerides). Le mélange est agité 5 minutes. La phase lipophile radiomarquée est recueillie.

Exemple 7 : Préparation automatisée du complexe $^{90}$Y-β- isopropyltropolone en solution dans le Lipiodol

On décrit en rapport avec la figure 1 la préparation automatisée du complexe $^{90}$Y-β- isopropyltropolone en solution dans le Lipiodol. Le système automatisé comprend un ensemble de fioles contenant les différents réactifs, ainsi que des réacteurs dans lesquels se produisent les réactions chimiques. La circulation des réactifs dans l'ensemble des fioles et réacteurs est commandée par un système de vannes (V1-V15) et de pompes (P1, P2). Le fonctionnement automatique des valves et des pompes est programmé et pré-enregistré dans un système de contrôle 4. Un automate convenant à la mise en oeuvre du procédé selon l'invention est du type de l'automate Taddeo (Comecer, Italie).
Brièvement, un volume de 0,5 mL de chlorure d'yttrium-90 ayant une radioactivité de 1,61 mCi et contenu dans un récipient A sont transférés dans le réacteur R. Un volume de 0,5 mL d'une solution β-isopropyltropolone à $10^{-2}$ mol/L dans l'éthanol dans un récipient B sont également transférés dans le réacteur R. Après 5 min à température ambiante, le milieu réactionnel est transféré sur une colonne 1 de type Sep-Pak® C$_{18}$ puis lavé par 5 mL d'eau distillée contenue dans la fiole F3. Le complexe $^{90}$Y-β- isopropyltropolone est ensuite élué dans le flacon C par 2,5 mL d'éthanol contenu dans la fiole F5. L'éthanol est évaporé à 100 °C et sous pression réduite, et 2 mL de Lipiodol, contenu dans la fiole F6, sont finalement ajoutés pour donner la composition radiomarquée désirée. Les déchets tels que les solutions de lavages et les réactifs en excès sont récupérés dans une cuve à déchets 2. Ces déchets sont d'abord filtrés par un système de filtration 3, avec d'être éliminés conformément aux bonnes pratiques de laboratoires.
Il est possible, afin d'améliorer la progression du mélange réactionnel, de faire traverser un mélange eau-éthanol, contenu dans la fiole F4, à travers la colonne. Ce lavage additionnel dépend de la lipophilie du ligand tropolone utilisé.
Rendement (Rdt) = 51,6 %
Pureté radiochimique (PRC) = 99,8 %
Comme on peut le constater au vu de ces résultats, le procédé automatisé selon l'invention permet d'obtenir une composition comprenant une phase organique lipophile dans laquelle est solubilisé un complexe de terre rare radioisotopique de manière sûre pour l'utilisateur, rapide et en obtenant une excellente pureté radiochimique.

Exemple 8 : Biodistribution du Lipiodol marqué par le complexe $^{90}$Y-[β-isopropyltropolone, 72 h post-injection

Un volume de 0,15 mL, ayant une radioactivité de 53 $\mu$Ci, de Lipiodol radiomarqué sont injectés dans l'artère hépatique de rats atteints d'un carcinome hépatocellulaire (injection de cellules N1S1 16 jours plus tôt). 72 h après injection, les rats sont euthanasiés et leurs organes pesés et comptés.

| Tissu testé | Activité injectée (%) | Activité injectée par gramme d'organe (%) |
|---|---|---|
| Tumeur | 30,2 | 13,4 |
| Foie tumoral | 17,1 | 7,6 |
| Foie sain | 22,2 | 4 |
| Poumons | 1,7 | 0,8 |
| Coeur | 0,3 | 0,3 |
| Rate | 0,4 | 0,9 |
| Reins | 4,7 | 2,6 |
| Estomac | 0,7 | 0,2 |
| Intestins | 2,2 | 0,2 |
| Os (fémur) | 1,8 | 1,7 |

Tel qu'on peut l'observer au vu des résultats de la figure 1, l'activité radioactive se retrouve principalement au niveau de la tumeur, du foie affecté et du foie sain. L'activité au niveau du foie sain reste toutefois trois fois plus faible qu'au niveau de la tumeur, indiquant une fixation préférentielle de la composition selon l'invention au niveau de la tumeur hépatique. La fixation au niveau des autres organes est également faible. La composition de Lipiodol radiomarqué grâce au complexe selon l'invention permet donc de cibler spécifiquement le foie et la tumeur hépatique. Les problèmes de fixation anarchique des complexes radioactifs sont donc évités grâce à la composition selon l'invention, le complexe [M(L)$_3$] étant particulièrement stable. La composition selon l'invention est donc plus sûre et très efficace.

**Revendications**

**1.** Composition thérapeutique comprenant un complexe de formule (1) suivante comprenant une terre rare radioisotopique, ledit complexe étant solubilisé dans une phase organique lipophile, ladite phase organique étant le Lipiodol®:

$$[M(L)_3] \qquad (1)$$

dans laquelle :

- M désigne la terre rare radioisotopique $^{90}$Y, et
- L désigne un ligand tropolone ou un ligand dérivé de la tropolone choisi parmi une halogénotropolone, l'a-methyltropolone, la β-méthyltropolone, la γ-méthyltropolone, l'α-isopropyltropolone, la β- isopropyltropolone, la γ-isopropyltropolone, la nootkatine, l'acide stipitatique, l'acide pubérrulique, l'acide pubérulonique, la purpurogalline, la colchicéine ou un dérivé de ceux-ci, L désignant de préférence la β- isopropyltropolone..

**2.** Composition selon la revendication 1 dans laquelle ledit Lipiodol® est mis en émulsion dans une phase aqueuse, de préférence ladite phase aqueuse étant du sérum physiologique.

**3.** Procédé de fabrication d'une composition thérapeutique selon l'une des revendications 1 à 2 comprenant les étapes de :

a. synthèse en une étape du complexe de formule (1),
b. purification du complexe de formule (1) sur colonne,
c. évaporation du solvant organique à une température comprise entre 40°C et 100°C,
d. filtration stérilisante,

e. solubilisation du complexe de formule (1) dans ladite phase organique lipophile.

4. Procédé selon la revendication 3 dans lequel la synthèse en une étape du complexe de formule (1) se fait à partir d'un précurseur ayant la formule (2) suivante :

$$MZ_3 \qquad (2)$$

dans laquelle M désigne une terre rare radioisotopique sous forme ionique et Z est un anion halogénure, un anion nitrate, un anion triflate ou un anion acétate.

5. Procédé selon la revendication 3 ou 4 comprenant en outre une étape finale de nanoencapsulation.

6. Composition selon l'une des revendications 1-2 pour une utilisation dans le cadre du traitement du cancer du foie chez l'humain.

**Patentansprüche**

1. Therapeutische Zusammensetzung, umfassend einen Komplex der folgenden Formel (1), der ein Seltenerdmetall-Radioisotop umfasst, wobei der Komplex in einer lipophilen organischen Phase solubilisiert ist, wobei es sich bei der organischen Phase um Lipiodol® handelt:

$$[M(L)_3] \qquad (1)$$

wobei:

- M für das Seltenerdmetall-Radioisotop $^{90}$Y steht und
- L für einen Tropolon-Liganden oder einen von Tropolon abgeleiteten Liganden, der aus einem Halogentropolon, $\alpha$-Methyltropolon, $\beta$-Methyltropolon, $\gamma$-Methyltropolon, $\alpha$-Isopropyltropolon, $\beta$-Isopropyltropolon, $\gamma$-Isopropyltropolon, Nootkatin, Stipitatsäure, Puberulsäure, Puberulonsäure, Purpurogallin, Colchicin oder einem Derivat davon ausgewählt ist, steht, wobei L vorzugsweise für $\beta$-Isopropyltropolon steht.

2. Zusammensetzung nach Anspruch 1, wobei das Lipiodol® in einer wässrigen Phase emulgiert ist, wobei es sich bei der wässrigen Phase vorzugsweise um physiologisches Serum handelt.

3. Verfahren zur Herstellung einer therapeutischen Zusammensetzung nach einem der Ansprüche 1 bis 2, das folgende Schritte umfasst:

a. Synthese des Komplexes der Formel (1) in einem Schritt,
b. Reinigung des Komplexes der Formel (1) an einer Säule,
c. Verdampfen des organischen Lösungsmittels bei einer Temperatur zwischen 40 °C und 100 °C,
d. Sterilfiltration,
e. Solubilisierung des Komplexes der Formel (1) in der lipophilen organischen Phase.

4. Verfahren nach Anspruch 3, wobei die Synthese des Komplexes der Formel (1) in einem Schritt ausgehend von einer Vorstufe mit der folgenden Formel (2) erfolgt:

$$MZ_3 \qquad (2)$$

wobei M für ein Seltenerdmetall-Radioisotop in ionischer Form steht und Z für ein Halogenid-Anion, ein Nitrat-Anion, ein Triflat-Anion oder ein Acetat-Anion steht.

5. Verfahren nach Anspruch 3 oder 4, das außerdem einen abschließenden Nanoverkapselungsschritt umfasst.

6. Zusammensetzung nach einem der Ansprüche 1-2 zur Verwendung im Rahmen der Behandlung von Leberkrebs beim Menschen.

**Claims**

1. Therapeutical composition, comprising a complex having the following formula (1) comprising a rare-earth radioisotope, said complex being solubilized in a lipophilic organic phase, said lipophilic organic phase being Lipiodol®:

$$[M(L)_3] \qquad (1)$$

wherein:

    - M designates the rare-earth radioisotope, and
    - L designates a tropolone ligand or a tropolone-derived ligand, L being chosen from amongst halogenotropolone, $\alpha$-methyltropolone, $\beta$-methyltropolone, $\gamma$-methyltropolone, $\alpha$-isopropyltropolone, $\beta$-isopropyltropolone, $\gamma$-isopropyltropolone, nootkatin, stipitatic acid, puberulic acid, puberulonic acid, purpurogallin, colchicine or a derivative of these substances, L preferably designating $\beta$-isopropyltropolone.

2. Composition according to one of the claims 1 to 4 wherein said lipophilic phase is put into emulsion in an aqueous phase, said aqueous phase preferably being a physiological serum.

3. Method of manufacture of a therapeutical composition according to one of the claims 1 to 2 comprising the steps of:

    a. one-step synthesis of the complex having formula (1),
    b. column purification of the complex having the formula (1),
    c. evaporation of the organic solvent at a temperature of 40°C to 100°C,
    d. sterilizing filtration,
    e. solubilizing the complex having the formula (1) in said lipophilic organic phase.

4. Method according to claim 3 wherein the one-step synthesis of the complex having formula (1) is done using a precursor having the following formula (2):

$$MZ_3 \qquad (2)$$

where M designates a rare-earth radioisotope and Z is a halogenic anion, a nitrate anion, a triflate anion or an acetate anion.

5. Method according to claim 3 or 4 also comprising a final step of nanoencapsulation.

6. Use of the composition according to one of the claims 1 to 2 for the treatment of liver cancer among humans.

Fig. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **E. GARIN ; P. BOURGUET.** Nuclear Medicine in Clinical Diagnosis and Treatment. Churchill-Livingstone, 2004, 473-483
- **B. LAMBERT.** *J.Nucl.Med.,* 2005, vol. 46, 60-66
- **S.J. WANG et al.** *J. Nucl. Med.,* 1996, vol. 37, 332-335
- **J. YU et al.** *Appl. Radiat. Isot.,* 2003, vol. 58, 567-573
- **P.Y. MU et al.** *J. Radioanal. Nucl. Chem.,* 2007, vol. 272, 669-671
- **S. SUBRAMANIAN et al.** *Cancer Bioth. Radiopharm.,* 2010, vol. 25, 539-543
- **T. DAS et al.** *Nucl. Med. Commun.,* 2009, vol. 30, 362-367
- **S.J. OH et al.** *Appl. Radiat. Isot.,* 2001, vol. 54, 419-427
- **S.J. OH et al.** *Appl. Radiat. Isot.,* 2003, vol. 59, 225-230
- **URIZZI et al.** Attachement of radiometals to LDL with lipid analogues of EDTA or DTPA type complexing agents. Comparison of two labeling procédures. *JOURNAL DE CHIMIE PHYSIQUE,* 01 Janvier 1997, vol. 94 (2), ISSN 0021-789, 371-375
- **JIANLIN Y et al.** Raman spectroscopic studies on tropolone complexes with La, Nd, Sm, Yb. *SPECTROCHIMICA ACTA. PART A : MOLECULAR AND BIOMOLECULAR SPECTROSCOPY,* 01 Juillet 2006, vol. 64 (4), ISSN 1386-1425, 1072-1076